(19) **Europäisches Patentamt / European Patent Office / Office européen des brevets**

(11) **EP 4 084 680 B1**

(12) **EUROPEAN PATENT SPECIFICATION**

(45) Date of publication and mention
of the grant of the patent:
**22.11.2023 Bulletin 2023/47**

(21) Application number: **21709522.3**

(22) Date of filing: **02.02.2021**

(51) International Patent Classification (IPC):
***A61B 5/021*** (2006.01)   ***A61B 5/00*** (2006.01)

(52) Cooperative Patent Classification (CPC):
**A61B 5/02108; A61B 5/7275;** A61B 5/0215;
A61B 2505/03; A61B 2505/05

(86) International application number:
**PCT/US2021/016246**

(87) International publication number:
**WO 2021/173311 (02.09.2021 Gazette 2021/35)**

(54) **HYPOTENSION PREDICTION WITH FEATURE TRANSFORMATION FOR ADJUSTABLE HYPOTENSION THRESHOLD**

HYPOTENSIONSVORHERSAGE MIT MERKMALTRANSFORMATION FÜR EINSTELLBARE HYPOTENSIONSSCHWELLE

PRÉDICTION D'HYPOTENSION À TRANSFORMATION DE CARACTÉRISTIQUES POUR SEUIL D'HYPOTENSION AJUSTABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.02.2020 US 202062981198 P**

(43) Date of publication of application:
**09.11.2022 Bulletin 2022/45**

(73) Proprietor: **Edwards Lifesciences Corporation**
**Irvine, CA 92614-5688 (US)**

(72) Inventors:
• **SCHNEIDER, Brennan, Michael**
**Irvine, CA 92614 (US)**
• **JIAN, Zhongping**
**Irvine, CA 92614 (US)**
• **AL HATIB, Feras**
**Irvine, CA 92614 (US)**
• **BUDDI, Sai, Prasad**
**Irvine, CA 92614 (US)**

(74) Representative: **Venner, Julia Ann et al**
**Kilburn & Strode LLP**
**Lacon London**
**84 Theobalds Road**
**London WC1X 8NL (GB)**

(56) References cited:
**US-A1- 2018 008 205**

**Description**

BACKGROUND

The present disclosure relates generally to arterial blood pressure monitoring, and more specifically to prediction of hypotension with an adjustable hypotension threshold.

Hypotension, or low blood pressure, can be a harbinger of serious medical complications, and even mortality, for patients undergoing surgery and those acutely or critically ill patients receiving treatment in an intensive care unit (ICU). The dangers associated with the occurrence of hypotension in a patient are due both to the potential injury caused by the hypotension itself and to the many serious underlying medical disorders that the occurrence of hypotension may signify.

In and of itself, hypotension in surgical patients or critically ill patients is a serious medical condition. For example, in the operating room (OR) setting, hypotension during surgery is associated with increased mortality and organ injury. Even short durations of extreme hypotension during surgery are associated with acute kidney injury and myocardial injury. Among critically ill patients, in-hospital mortality may be nearly doubled for patients experiencing hypotension after emergency intubation. For surgical patients and seriously ill patients alike, hypotension, if not corrected, can impair organ perfusion, resulting in irreversible ischemic damage, neurological deficit, cardiomyopathy, and renal impairment.

In addition to posing serious risks to surgical patients and critically ill patients in its own right, hypotension can be a symptom of one or more other serious underlying medical conditions. Examples of underlying conditions for which hypotension may serve as an acute symptom include sepsis, myocardial infarction, cardiac arrhythmia, pulmonary embolism, hemorrhage, dehydration, anaphylaxis, acute reaction to medication, hypovolemia, insufficient cardiac output, and vasodilatory shock. Due to its association with such a variety of serious medical conditions, hypotension is relatively common, and is often seen as one of the first signs of patient deterioration in the OR and ICU.

Conventional patient monitoring for hypotension in the OR and ICU settings can include continuous or periodic blood pressure measurement. However, such monitoring, whether continuous or periodic, typically provides no more than a real-time assessment. As a result, hypotension in a surgical patient or critically ill patient is usually detected only after it begins to occur, so that remedial measures and interventions are not initiated until the patient has entered a hypotensive state. Although, as noted above, extreme hypotension can have potentially devastating medical consequences quite quickly, even relatively mild levels of hypotension can herald or precipitate cardiac arrest in patients with limited cardiac reserve. US 2018/0008205 A1 discloses a predictive weighting of hypotension profiling parameters.

In view of the frequency with which hypotension is observed to occur in the OR and ICU settings, and due to the serious and sometimes immediate medical consequences that can result when it does occur, a solution enabling prediction of a future hypotension event, before its occurrence, is highly desirable.

SUMMARY

In one example, a method for monitoring of arterial pressure of a patient and providing a warning to medical personnel of a predicted future hypotension event of the patient includes receiving, by a hemodynamic monitor, sensed hemodynamic data representative of an arterial pressure waveform of the patient. The method further includes performing, by the hemodynamic monitor, waveform analysis of the hemodynamic data to determine a plurality of hypotension profiling parameters predictive of a future hypotension event for the patient, and generating, by the hemodynamic monitor, a set of transformed hypotension profiling parameters. Each transformed hypotension profiling parameter is a function of a corresponding one of the plurality of hypotension profiling parameters at a standard mean arterial pressure (MAP) threshold for hypotension, a mean of the corresponding one of the plurality of hypotension profiling parameters at the standard MAP threshold for hypotension, a standard deviation of the corresponding one of the plurality of hypotension profiling parameters at the standard MAP threshold, a mean of the corresponding one of the plurality of hypotension profiling parameters at an adjusted MAP threshold for hypotension, and a standard deviation of the corresponding one of the plurality of hypotension profiling parameters at the adjusted MAP threshold for hypotension. The method further includes determining, by the hemodynamic monitor based on the set of transformed hypotension profiling parameters, a risk score representing a probability of the future hypotension event for the patient, and invoking, by the hemodynamic monitor, a sensory alarm to produce a sensory signal in response to the risk score satisfying a predetermined risk criterion.

In another example, a system for monitoring of arterial pressure of a patient and providing a warning to medical personnel of a predicted future hypotension event includes a hemodynamic sensor, a system memory, a user interface, and a hardware processor. The hemodynamic sensor produces hemodynamic data representative of an arterial pressure waveform of the patient. The system memory stores hypotension prediction software code including a predictive weighting module. The user interface includes a sensory alarm that provides a sensory signal to warn the medical personnel of the predicted future hypotension event prior to the patient entering a hypotensive state. The hardware processor is configured to execute the hypotension prediction software code to perform waveform analysis of the hemodynamic data to determine a plurality of hypotension profiling parameters predictive of a future hypotension event for the patient, and to generate a set of transformed hypotension profiling parameters. Each transformed hypotension profiling parameter is a function of a corresponding one of the plurality of hypotension profiling parameters at a standard mean arterial pressure (MAP) threshold for hypotension, a mean of the corresponding one of the plurality of hypotension profiling parameters at the standard MAP threshold for hypotension, a standard deviation of the corresponding one of the plurality of hypotension profiling parameters at the standard MAP threshold, a mean of the corresponding one of the plurality of hypotension profiling parameters at an adjusted MAP threshold for hypotension, and a standard deviation of the corresponding one of the plurality of hypotension profiling parameters at the adjusted MAP threshold for hypotension. The hardware processor is further configured to execute the hypotension prediction software code to determine, based on the set of transformed hypotension profiling parameters, a risk score representing a probability of the future hypotension event for the patient, and invoke the sensory alarm of the user interface in response to the risk score satisfying a predetermined risk criterion.

BRIEF DESCRIPTION OF THE DRAWINGS

FIG. 1 is a perspective view of an example hemodynamic monitor that determines a risk score representing a probability of a future hypotension event for a patient.
FIG. 2 is a perspective view of an example minimally invasive pressure sensor for sensing hemodynamic data representative of arterial pressure of a patient.
FIG. 3 is a perspective view of an example non-invasive sensor for sensing hemodynamic data representative of arterial pressure of a patient.
FIG. 4 is a block diagram illustrating an example hemodynamic monitoring system that determines a risk score representing a probability of a future hypotension event for a patient based on a set of transformed hypotension profiling parameters.
FIG. 5 is a graph illustrating an example trace of an arterial pressure waveform including example indicia corresponding to the probability of future hypotension in a patient.
FIG. 6 is a flow diagram illustrating example operations of the hemodynamic monitoring system to generate a set of transformed hypotension profiling parameters and determine a risk score representing a probability of a future hypotension event using the set of transformed hypotension profiling parameters.

DETAILED DESCRIPTION

As described herein, a hemodynamic monitoring system implements a predictive risk model that produces a risk score representing a probability of a future hypotension event for a patient. The risk score is determined based on hypotension profiling parameters that are predictive of the future hypotension event. Risk coefficients that implement the weighting are selected based on a standard (or defined) mean arterial pressure (MAP) threshold for hypotension, such as a pressure of 65 millimeters of Mercury (mmHg) or other defined pressure threshold. The selection of risk coefficients and/or hypotension profiling parameters can be accomplished via training (e.g., offline training) of the predictive risk model using machine learning or other techniques to minimize a cost function representing the error of the predictive risk model output to the true value of training subsets that define hypotension according to the standard MAP threshold for hypotension.

According to techniques of this disclosure, the hemodynamic monitoring system can utilize an adjustable MAP threshold for hypotension to represent a modified hypotension pressure threshold. Rather than modify the predictive risk model (via retraining or otherwise) to accommodate the adjustable (e.g., user defined or otherwise adjusted) MAP threshold, the hemodynamic monitoring system generates a set of transformed hypotension profiling parameters. Each transformed hypotension profiling parameter is a function of the hypotension profiling parameters, and mean and standard deviation values of the hypotension profiling parameters at the standard MAP threshold for hypotension and at the adjusted MAP threshold. That is, rather than require retraining or other modification of the predictive risk model to determine new risk coefficients based on the modified definition of hypotension (i.e., the adjusted MAP threshold), the hemodynamic monitoring system adjusts the hypotension profiling parameters (or features) extracted from the hemodynamic data to accommodate the adjusted MAP threshold for hypotension. A risk score representing the probability of a future hypotension event for the patient is determined based on the set of transformed hypotension profiling parameters.

Accordingly, a hemodynamic monitoring system implementing techniques of this disclosure can utilize an adjustable pressure threshold for hypotension without requiring retraining or other modifications to the predictive risk model, thereby enabling real-time updates to the hypotension threshold during operation in, e.g., an operating room (OR), an intensive care unit (ICU), or other patient care environment. As such, the system can provide a risk score representing a probability of future hypotension of the patient to enable timely and effective intervention while also taking advantage of the training and/or experience of medical personnel that could warrant the use of a modified hypotension threshold, thereby increasing usability of the system by the medical personnel for patient care.

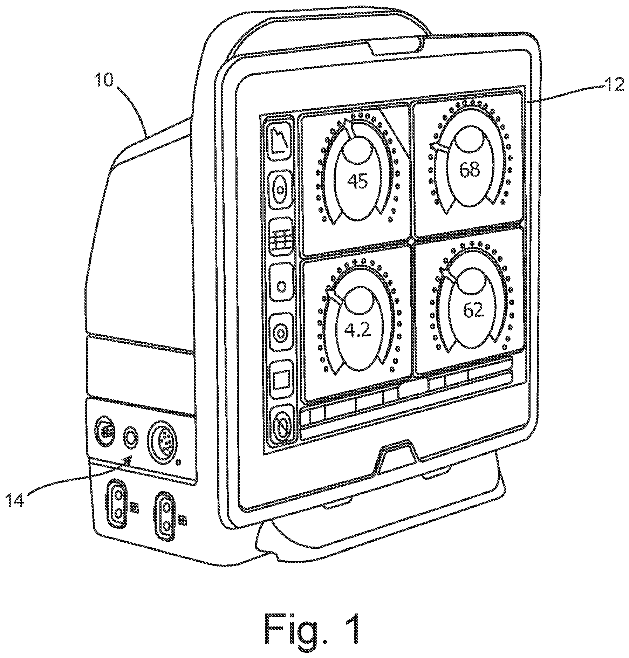

FIG. 1 is a perspective view of hemodynamic monitor 10 that determines a risk score representing a probability of a future hypotension event for a patient. As illustrated in FIG. 1, hemodynamic monitor 10 includes display 12 that, in the example of FIG. 1, presents a graphical user interface including control elements (e.g., graphical control elements) that enable user interaction with hemodynamic monitor 10. Hemodynamic monitor 10 can also include a plurality of input and/or output (I/O) connectors configured for wired connection (e.g., electrical and/or communicative connection) with one or more peripheral components, such as one or more hemodynamic sensors, as is further described below. For instance, as illustrated in FIG. 1, hemodynamic monitor 10 can include I/O connectors 14. While the example of FIG. 1 illustrates five separate I/O connectors 14, it should be understood that in other examples, hemodynamic monitor 10 can include fewer than five I/O connectors or greater than five I/O connectors. In yet other examples, hemodynamic monitor 10 may not include I/O connectors 14, but rather may communicate wirelessly with various peripheral devices.

As is further described below, hemodynamic monitor 10 includes one or more processors and computer-readable memory that stores hypotension prediction software code which is executable to produce a risk score representing a probability of a future hypotension event for a patient. For example, hemodynamic monitor 10 can receive sensed hemodynamic data representative of an arterial pressure waveform of the patient, such as via one or more hemodynamic sensors connected to hemodynamic monitor 10 via I/O connectors 14. Hemodynamic monitor 10 executes the hypotension prediction software code to obtain, using the received hemodynamic data, multiple hypotension profiling parameters (e.g., features), which can include one or more vital sign parameters characterizing vital sign data of the patient, as well as differential and combinatorial parameters derived from the one or more vital sign parameters, as is further described below.

As described herein, hemodynamic monitor 10 can further utilize an adjusted MAP threshold for hypotension, the adjusted MAP threshold representing a deviation from the standard MAP threshold from which the coefficients utilized by the hypotension prediction software code are determined. For instance, hemodynamic monitor 10 can present graphical control elements (e.g., at a graphical user interface presented at display 12) that enable user input of an adjusted MAP threshold for hypotension, though inputs received via physical controls (e.g., buttons, knobs, or other physical input controls) are possible.

For example, as illustrated in FIG. 1, hemodynamic monitor 10 can present a graphical user interface at display 12. Display 12 can be a liquid crystal display (LCD), a light-emitting diode (LED) display, an organic light-emitting diode (OLED) display, or other display device suitable for providing information to users in graphical form. In some examples, such as the example of FIG. 1, display 12 can be a touch-sensitive and/or presence-sensitive display device configured to receive user input in the form of gestures, such as touch gestures, scroll gestures, zoom gestures, swipe gestures, or other gesture input. Hemodynamic monitor 10 presents control elements that enable user input of an adjusted MAP threshold, such as an absolute pressure (e.g., a MAP threshold value), a deviation value (e.g., a deviation from the standard MAP threshold), or other indication of an adjusted MAP threshold that can be, in some examples, user defined, such as by medical personnel.

In response to receiving the adjusted MAP threshold, hemodynamic monitor 10 executes the hypotension prediction software code to generate a set of transformed hypotension profiling parameters. As is further described below, each transformed hypotension profiling parameters is a function of a corresponding one of the plurality of hypotension profiling parameters at a standard mean arterial pressure (MAP) threshold for hypotension, a mean of the corresponding one of the plurality of hypotension profiling parameters at the standard MAP threshold for hypotension, a standard deviation of the corresponding one of the plurality of hypotension profiling parameters at the standard MAP threshold, a mean of the corresponding one of the plurality of hypotension profiling parameters at an adjusted MAP threshold for hypotension, and a standard deviation of the corresponding one of the plurality of hypotension profiling parameters at the adjusted MAP threshold for hypotension.

Hemodynamic monitor 10 executes the hypotension prediction software code to apply a plurality of risk coefficients to the set of transformed hypotension profiling parameters to produce a weighted combination resulting in the risk score that represents the probability of a future hypotension event for the patient. The plurality of risk coefficients, as described in further detail below, can be determined based on the standard MAP threshold, such as 65 mmHg, or other defined pressure threshold. As such, rather than require retraining of the prediction model to identify new coefficients based on the adjusted MAP threshold, hemodynamic monitor 10 executes the hypotension prediction software code to determine the risk score by applying the risk coefficients that were determined based on the standard MAP threshold to the set of transformed hypotension profiling parameters, thereby enabling dynamic adaptation of the model to an adjusted MAP threshold that may be based on training and expertise of medical personnel.

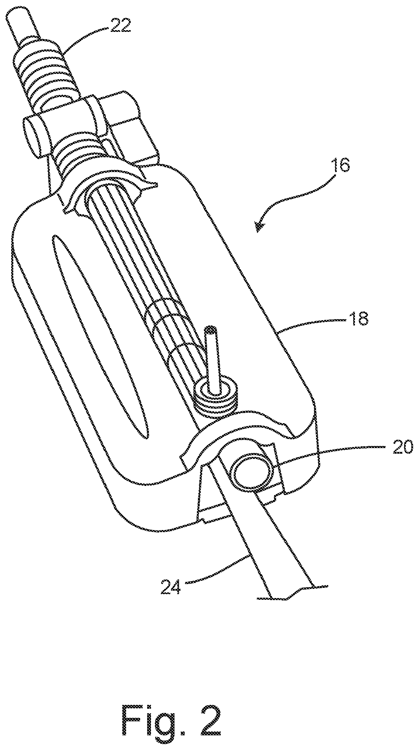

FIG. 2 is a perspective view of hemodynamic sensor 16 that can be attached to a patient for sensing hemodynamic data representative of arterial pressure of the patient. Hemodynamic sensor 16, illustrated in FIG. 2, is one example of a minimally invasive hemodynamic sensor that can be attached to the patient via, e.g., a radial arterial catheter inserted into an arm of the patient. In other examples, hemodynamic sensor 16 can attached to the patient via a femoral arterial catheter inserted into a leg of the patient.

As illustrated in FIG. 2, hemodynamic sensor 16 includes housing 18, fluid input port 20, catheter-side fluid port 22, and I/O cable 24. Fluid input port 20 is configured to be connected via tubing or other hydraulic connection to a fluid source, such as a saline bag or other fluid input source. Catheter-side fluid port 22 is configured to be connected via tubing or other hydraulic connection to a catheter (e.g., a radial arterial catheter or a femoral arterial catheter) that is inserted into an arm of the patient (i.e., a radial arterial catheter) or a leg of the patient (i.e., a femoral arterial catheter). I/O cable 24 is configured to connect to hemodynamic monitor 10 via, e.g., one or more of I/O connectors 14 (FIG. 1). Housing 18 of hemodynamic sensor 16 encloses one or more pressure transducers, communication circuitry, processing circuity, and corresponding electronic components to sense fluid pressure corresponding to arterial pressure of the patient that is transmitted to hemodynamic monitor 10 (FIG. 1) via I/O cable 24.

In operation, a column of fluid (e.g., saline solution) is introduced from a fluid source (e.g., a saline bag) through hemodynamic sensor 16 via fluid input port 20 to catheter-side fluid port 22 toward the catheter inserted into the patient. Arterial pressure is communicated through the fluid column to pressure sensors located within housing 16 which sense the pressure of the fluid column. Hemodynamic sensor 16 translates the sensed pressure of the fluid column to an electrical signal via the pressure transducers and outputs the corresponding electrical signal to hemodynamic monitor 10 (FIG. 1) via I/O cable 24. Hemodynamic sensor 16 therefore transmits analog sensor data (or a digital representation of the analog sensor data) to hemodynamic monitor 10 (FIG. 1) that is representative of substantially continuous beat-to-beat monitoring of the arterial pressure of the patient.

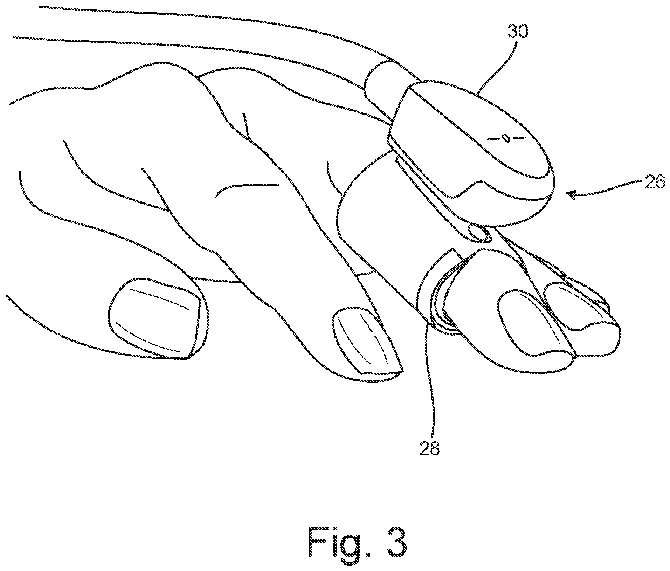

FIG. 3 is a perspective view of hemodynamic sensor 26 for sensing hemodynamic data representative of arterial pressure of a patient. Hemodynamic sensor 26, illustrated in FIG. 3, is one example of a non-invasive hemodynamic sensor that can be attached to the patient via one or more finger cuffs to sense data representative of arterial pressure of the patient. As illustrated in FIG. 3, hemodynamic sensor 26 includes inflatable finger cuff 28 and heart reference sensor 30. Inflatable finger cuff 28 includes an inflatable blood pressure bladder configured to inflate and deflate as controlled by a pressure controller (not illustrated) that is pneumatically connected to inflatable finger cuff 28. Inflatable finger cuff 28 also includes an optical (e.g., infrared) transmitter and an optical receiver that are electrically connected to heart reference sensor 30 to measure the changing volume of the arteries in the finger.

In operation, the pressure controller continually adjusts pressure within the finger cuff to maintain a constant volume of the arteries in the finger (i.e., the unloaded volume of the arteries) as measured by heart reference sensor 30 via the optical transmitter and optical receiver of inflatable finger cuff 28. The pressure applied by the pressure controller to continuously maintain the unloaded volume is representative of the blood pressure in the finger, and is communicated by the pressure controller to heart reference sensor 30. Heart reference sensor 30 translates the pressure signal representative of the blood pressure in the finger to hemodynamic data representative of the arterial pressure waveform of the patient, which is transmitted to hemodynamic monitor 10 (FIG. 1) via, e.g., I/O connectors 14 (FIG. 1). Accordingly, hemodynamic sensor 26 transmits sensor data that is representative of substantially continuous beat-to-beat monitoring of the arterial pressure of the patient.

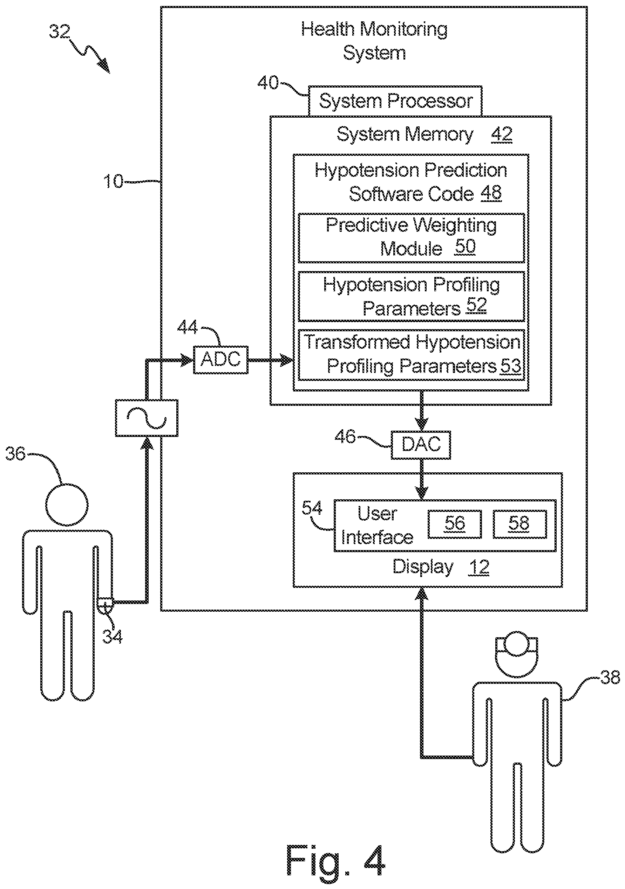

FIG. 4 is a block diagram of hemodynamic monitoring system 32 that determines a risk score representing a probability of a future hypotension event based on a set of transformed hypotension profiling parameters. As illustrated in FIG. 4, hemodynamic monitoring system 32 includes hemodynamic monitor 10 and hemodynamic sensor 34. Hemodynamic monitoring system 32 can be implemented within a patient care environment, such as an ICU, an OR, or other patient care environment. As illustrated in FIG. 4, the patient care environment can include patient 36 and healthcare worker 38 trained to utilize hemodynamic monitoring system 32.

Hemodynamic monitor 10, as described above with respect to FIG. 1, can be, e.g., an integrated hardware unit including system processor 40, system memory 42, display 12, analog-to-digital (ADC) converter 44, and digital-to-analog (DAC) converter 46. In other examples, any one or more components and/or described functionality of hemodynamic monitor 10 can be distributed among multiple hardware units. For instance, in some examples, display 12 can be a separate display device that is remote from and operatively coupled with hemodynamic monitor 10. In general, though illustrated and described in the example of FIG. 4 as an integrated hardware unit, it should be understood that hemodynamic monitor 10 can include any combination of devices and components that are electrically, communicatively, or otherwise operatively connected to perform functionality attributed herein to hemodynamic monitor 10.

As illustrated in FIG. 4, system memory 42 stores hypotension prediction software code 48. Hypotension prediction software code 48 includes predictive weighting module 50, hypotension profiling parameters 52, and transformed hypotension profiling parameters 53. Display 12 provides user interface 54, which includes control elements 56 that enable user interaction with hemodynamic monitor 10 and/or other components of hemodynamic monitoring system 32. User interface 54, as illustrated in FIG. 4, also provides sensory alarm 58 to provide warning to medical personnel of a predicted future hypotension event of patient 36, as is further described below.

Hemodynamic sensor 34 can be attached to patient 36 to sense hemodynamic data representative of an arterial pressure waveform of patient 36. Hemodynamic sensor 34 is operatively connected to hemodynamic monitor 10 (e.g.,

electrically and/or communicatively connected via wired or wireless connection, or both) to provide the sensed hemodynamic data to hemodynamic monitor 10. In some examples, hemodynamic sensor 34 provides the hemodynamic data representative of the arterial pressure waveform of patient 36 to hemodynamic monitor 10 as an analog signal, which is converted by ADC 44 to digital hemodynamic data representative of the arterial pressure waveform. In other examples, hemodynamic sensor 34 can provide the sensed hemodynamic data to hemodynamic monitor 10 in digital form, in which case hemodynamic monitor 10 may not include or utilize ADC 44. In yet other examples, hemodynamic sensor 34 can provide the hemodynamic data representative of the arterial pressure waveform of patient 36 to hemodynamic monitor 10 as an analog signal, which is analyzed in its analog form by hemodynamic monitor 10.

Hemodynamic sensor 34 can be a non-invasive or minimally invasive sensor attached to patient 36. For instance, hemodynamic sensor 34 can take the form of minimally invasive hemodynamic sensor 16 (FIG. 2), non-invasive hemodynamic sensor 26 (FIG. 3), or other minimally invasive or non-invasive hemodynamic sensor. In some examples, hemodynamic sensor 34 can be attached non-invasively at an extremity of patient 36, such as a wrist, an arm, a finger, an ankle, a toe, or other extremity of patient 36. As such, hemodynamic sensor 34 can take the form of a small, lightweight, and comfortable hemodynamic sensor suitable for extended wear by patient 36 to provide substantially continuous beat-to-beat monitoring of the arterial pressure of patient 36 over an extended period of time, such as minutes or hours.

In certain examples, hemodynamic sensor 34 can be configured to sense an arterial pressure of patient 36 in a minimally invasive manner. For instance, hemodynamic sensor 34 can be attached to patient 36 via a radial arterial catheter inserted into an arm of patient 36. In other examples, hemodynamic sensor 34 can be attached to patient 36 via a femoral arterial catheter inserted into a leg of patient 36. Such minimally invasive techniques can similarly enable hemodynamic sensor 34 to provide substantially continuous beat-to-beat monitoring of the arterial pressure of patient 36 over an extended period of time, such as minutes or hours.

System processor 40 is configured to execute hypotension prediction software code 48, which implements predictive weighting module 50 utilizing hypotension profiling parameters 52 and transformed hypotension profiling parameters 53 to produce a risk score representing a probability of a future hypotension event for patient 36. Examples of system processor 40 can include any one or more of a microprocessor, a controller, a digital signal processor (DSP), an application specific integrated circuit (ASIC), a field-programmable gate array (FPGA), or other equivalent discrete or integrated logic circuitry.

System memory 42 can be configured to store information within hemodynamic monitor 10 during operation. System memory 42, in some examples, is described as computer-readable storage media. In some examples, a computer-readable storage medium can include a non-transitory medium. The term "non-transitory" can indicate that the storage medium is not embodied in a carrier wave or a propagated signal. In certain examples, a non-transitory storage medium can store data that can, over time, change (e.g., in RAM or cache). System memory 42 can include volatile and non-volatile computer-readable memories. Examples of volatile memories can include random access memories (RAM), dynamic random access memories (DRAM), static random access memories (SRAM), and other forms of volatile memories. Examples of non-volatile memories can include, e.g., magnetic hard discs, optical discs, flash memories, or forms of electrically programmable memories (EPROM) or electrically erasable and programmable (EEPROM) memories.

Display 12 can be a liquid crystal display (LCD), a light-emitting diode (LED) display, an organic light-emitting diode (OLED) display, or other display device suitable for providing information to users in graphical form. User interface 54 can include graphical and/or physical control elements that enable user input to interact with hemodynamic monitor 10 and/or other components of hemodynamic monitoring system 32. In some examples, user interface 54 can take the form of a graphical user interface (GUI) that presents graphical control elements presented at, e.g., a touch-sensitive and/or presence sensitive display screen of display 12. In such examples, user input can be received in the form of gesture input, such as touch gestures, scroll gestures, zoom gestures, or other gesture input. In certain examples, user interface 54 can take the form of and/or include physical control elements, such as a physical buttons, keys, knobs, or other physical control elements configured to receive user input to interact with components of hemodynamic monitoring system 32.

In operation, hemodynamic sensor 34 senses hemodynamic data representative of an arterial pressure waveform of patient 36. Hemodynamic sensor 34 provides the hemodynamic data (e.g., as analog sensor data), to hemodynamic monitor 10. ADC 44 converts the analog hemodynamic data to digital hemodynamic data representative of the arterial pressure waveform of the patient.

System processor 40 executes hypotension prediction software code 48 to determine, using the received hemodynamic data, a risk score representing a probability of a future hypotension event for patient 36. For instance, system processor 40 can execute hypotension prediction software code 48 to obtain, using the received hemodynamic data, multiple hypotension profiling parameters 52. Hypotension profiling parameters 52 can include one or more vital sign parameters characterizing vital sign data of patient 36, as well as differential and combinatorial parameters derived from the one or more vital sign parameters, as is further described below. As is further described below, system processor 40 further executes hypotension prediction software code 48 to generate the set of transformed hypotension profiling parameters 53 based on an adjusted MAP threshold for hypotension.

Predictive weighting module 50 of hypotension prediction software code 48 determines a risk score corresponding to the probability of a future hypotension event for patient 36 based on a weighted combination of transformed hypotension profiling parameters 53. That is, predictive weighting module 50 applies a plurality of risk coefficients stored at system memory 42 to transformed hypotension profiling parameters 53 to produce the weighted combination resulting in the risk score.

The risk coefficients can be determined via training operations (e.g., offline training) using machine learning or other techniques to minimize a cost function that represents the error of the risk score to the true value of training subsets (e.g., aggregations of data from multiple patients) that define hypotension according to a standard MAP threshold for hypotension. That is, risk coefficients utilized by predictive weighting module 50 can be selected via training operations to minimize the error of the predictive risk score determined by hypotension prediction software code 48 as predictive of a future hypotension event using hypotension profiling parameters 52. The error of the predictive risk score to predict future hypotension events can be evaluated with respect to positive and negative training data subsets that define the occurrence of hypotension with respect to a standard (e.g., defined) MAP threshold, such as 65 mmHg or other pressure thresholds.

As described herein, hemodynamic monitor 10 can receive an adjusted MAP threshold for hypotension, such as a user defined MAP threshold via control elements 56 of user interface 54. The adjusted MAP threshold can represent a deviation from the standard MAP threshold by which risk coefficients utilized by predictive weighting module 50 are determined. The adjusted MAP threshold provided by, e.g., healthcare worker 38, can take the form of an absolute pressure (e.g., a MAP threshold value), a deviation value (e.g., a deviation from the standard MAP threshold), or other indication of an adjusted MAP threshold.

Hypotension prediction software code 48, in response to receiving the adjusted MAP threshold, generates the set of transformed hypotension profiling parameters 53 using hypotension profiling parameters 52. As is further described below, each transformed hypotension profiling parameter of transformed hypotension profiling parameters 53 can be a function of a corresponding one of hypotension profiling parameters 52 (i.e., corresponding to a standard MAP threshold for hypotension), a mean of the corresponding one of hypotension profiling parameters 52 at the standard MAP threshold for hypotension, a standard deviation of the corresponding one of hypotension profiling parameters 52 at the standard MAP threshold, a mean of the corresponding one of hypotension profiling parameters 52 at the adjusted MAP threshold for hypotension, and a standard deviation of the corresponding one of hypotension profiling parameters 52 at the adjusted MAP threshold for hypotension.

System processor 40 executes hypotension prediction software code 48 to determine the predictive risk score as a weighted combination of transformed hypotension profiling parameters 53 using the risk coefficients that were determined based on the standard MAP threshold. As such, hypotension prediction software code 48 dynamically adapts to determine the risk score using transformed hypotension profiling parameters 53 without requiring retraining or other modifications to the prediction model to identify new coefficients.

System processor 40 further executes hypotension prediction software code 48 to invoke sensory alarm 58 via user interface 54 in response to determining that the risk score satisfies predetermined risk criteria, as is further described below. For example, hypotension prediction software code 48 can invoke sensory alarm 58 to warn of a hypotension event predicted to occur, e.g., one to five minutes in the future, or up to approximately thirty minutes in the future. Sensory alarm 58 can be implemented as one or more of a visual alarm, an audible alarm, a haptic alarm, or other type of sensory alarm. For instance, sensory alarm 58 can be invoked as any combination of flashing and/or colored graphics shown by use interface 54 on display 12, display of the risk score via user interface 54 on display 12, a warning sound such as a siren or repeated tone, and a haptic alarm configured to cause hemodynamic monitor 10 to vibrate or otherwise deliver a physical impulse perceptible to healthcare worker 38 or other user.

Accordingly, hemodynamic monitor 10 provides a warning to medical personnel of a predicted future hypotension event of patient 36, thereby enabling timely and effective intervention to prevent the predicted future hypotension event. Moreover, rather than require retraining of the predictive risk model to determine new risk coefficients based on an adjusted MAP threshold, hemodynamic monitor 10, implementing techniques of this disclosure, generates a set of transformed hypotension profiling parameters (or features) that are used to determine the risk score via application of the unmodified risk coefficients. As such, hemodynamic monitor 10 enables real-time updates by medical personnel to the MAP threshold defining hypotension, thereby increasing the usability of hemodynamic monitor 10 via dynamic adaptation to, e.g., user defined changes that may be based on training and expertise of attending medical personnel to predict a future hypotension event for patient 36.

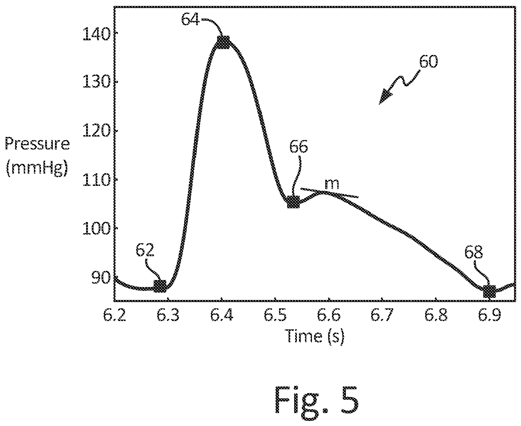

FIG. 5 is a graph illustrating an example trace of arterial pressure waveform 60 corresponding to hemodynamic data sensed by hemodynamic sensor 34 and received by hemodynamic monitor 10. As illustrated in FIG. 5, hemodynamic waveform 60 (e.g., represented via digital hemodynamic data) can include various indicia predictive of a future hypotension event for patient 36. FIG. 5 illustrates example indicia 62, 64, 66, and 68, corresponding respectively to the start of a heart beat (indicium 62), the maximum systolic pressure marking the end of systolic rise (indicium 64), the presence of the dicrotic notch marking the end of systolic decay (indicium 66), and the diastole of the heartbeat (indicium 68) of patient 36. Also shown in FIG. 5 is example slope "m" of adjusted arterial pressure waveform 60, though it should be understood that slope "m" is merely representative of multiple slopes that may be determined at multiple locations along arterial pressure waveform 60.

Additional indicia predictive of future hypotension for patient 36 can be extracted from hemodynamic waveform 60 by hypotension prediction software code 48 based on behavior of hemodynamic waveform 60 in various intervals, such as in the interval from the maximum systolic pressure at indicium 64 to the diastole at indicium 66, as well as the interval from the start of the heartbeat at indicium 62 to the diastole at indicium 66. The behavior of arterial pressure waveform 60 during intervals: 1) systolic rise 62-64, 2) systolic decay 64-66, 3) systolic phase 62-66, 4) diastolic phase 66-68, 5) interval 64-68, and 6) heartbeat interval 62-68, can be determined by hypotension prediction software code 48 by determining the area under the curve of hemodynamic waveform 60 and the standard deviation of hemodynamic waveform 60 in each of intervals 1-6. The respective areas and standard deviations determined for intervals 1-6 can serve as additional indicia predictive of future hypotension for patient 36.

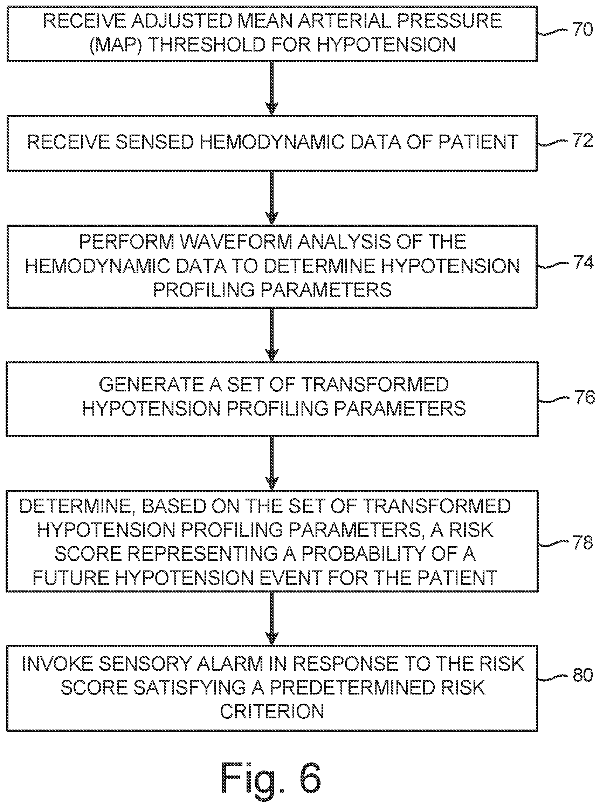

FIG. 6 is a flow diagram illustrating example operations to generate a set of transformed hypotension profiling parameters and determine a risk score representing a probability of a future hypotension event using the set of transformed hypotension profiling parameters. For purposes of clarity and ease of discussion, the example operations are described below within the context of hemodynamic monitoring system 32 of FIG. 4.

An adjusted MAP threshold for hypotension is received by hemodynamic monitor 10 (Step 70). For example, hemodynamic monitor 10 can receive an adjusted MAP threshold for hypotension provided by, e.g., healthcare worker 38 via control elements 56 of user interface 54. Hemodynamic monitor 10 receives sensed hemodynamic data representative of an arterial pressure waveform of patient 36 (Step 72). For instance, hemodynamic monitor 10 can receive an analog hemodynamic sensor signal representative of an arterial pressure waveform of patient 36 from hemodynamic sensor 34.

Hemodynamic monitor 10 performs waveform analysis of the hemodynamic data to determine a plurality of hypotension profiling parameters predictive of a future hypotension event for patient 36 (Step 74). For example, hemodynamic monitor 10 can execute hypotension prediction software code 48 to perform waveform analysis of the hemodynamic data to obtain hypotension profiling parameters 52 that are predictive of future hypotension in patient 36. Hypotension profiling parameters 52 can include one or more of vital sign parameters characterizing vital sign data of patient 36, differential parameters derived from the vital sign parameters, and combinatorial parameters representing combinations of one or more of the vital sign parameters and differential parameters.

Vital sign parameters characterizing vital sign data can include, e.g., stroke volume, heart rate, respiration, cardiac contractility, mean arterial pressure, baroreflex sensitivity measures, hemodynamic complexity measures, frequency domain hemodynamic features, or other vital sign parameters. Baroreflex sensitivity measures quantify the relationship between complementary physiological processes. For example, a decrease in blood pressure in a healthy patient is typically compensated by an increase in heart rate and/or an increase in peripheral resistance. The baroreflex sensitivity measures that may be included in the one or more vital sign parameters characterizing vital sign data correspond to the degree to which patient 36 is responding appropriately to normal physiological variations.

Hemodynamic complexity measures quantify the amount of regularity in cardiac measurements over time, as well as the entropy, e.g., the unpredictability of fluctuations in cardiac measurements over time. For instance, unpredictable cardiac fluctuations are a normal phenomenon associated with health. Very low entropy, i.e., a high degree of regularity in cardiac measurements over time and the substantial absence of unpredictable fluctuations, can be a significant warning sign of an impending hypotension event. Frequency domain hemodynamic features quantify various measures of cardiac performance as a function of frequency rather than time.

Hypotension predication software code 48 can further determine differential parameters based on the one or more vital sign parameters characterizing vital sign data of patient 36. Hypotension prediction software code 48 can derive the differential parameters from the one or more vital sign parameters by determining variations of the one or more vital sign parameters with respect to time, with respect to frequency, or with respect to other parameters from among the one or more vital sign parameters. As a result, each of one or more vital sign parameters can give rise to one, two, or several differential parameters included among hypotension profiling parameters 52.

For example, the differential parameter stroke volume variation (SVV) can be derived based on changes in the parameter stroke volume (SV) as a function of time and/or as a function of sampling frequency. Similarly, changes in mean arterial pressure ($\Delta$MAP) can be derived as a differential parameter with respect to time and/or sampling frequency. As a further example, changes in MAP with respect to time can be derived by subtracting the average of the MAP over the past five minutes, ten minutes, or other time durations, from the current value of the MAP.

Hypotension prediction software code 48 can generate combinatorial parameters included in hypotension profiling parameters 52 using the one or more vital sign parameters and the derived differential parameters. For example, the combinatorial parameters can be generated using the one or more vital sign parameters and the differential parameters by generating a power combination of a subset of the one or more vital sign parameters and the differential parameters. For instance, each of the combinatorial parameters can be generated as a power combination of three parameters, which can be randomly or purposefully selected, from among the one or more vital sign parameters characterizing vital sign data and/or the differential parameters. Each of the three parameters selected from among the one or more vital sign parameters and/or the differential parameters can be raised to an exponential power, and can be multiplied with or added to the other two parameters analogously raised to an exponential power. The exponential power to which each of the three parameters selected from the one or more vital sign parameters and/or the differential parameters is raised can be, but need not be, the same exponential power.

In some examples, generation of the combinatorial parameters can be performed using a predetermined and limited integer range of exponential powers. For instance, the exponential powers used to generate the combinatorial parameters can be integer powers selected from among negative two, negative one, zero, one, and two (-2, -1, 0, 1, 2). As such, each combinatorial parameter can be expressed, in some examples, according to the following equation:

$$X = Y_1^a * Y_2^b * ... Y_n^c \qquad \text{(Equation 1)}$$

where Y is one of one or more vital sign parameters characterizing vital sign data or one of the differential parameters, $n$ is any integer greater than two, and each of $a, b,$ and $c$ can be any one of -2, -1, 0, 1, and 2. In some examples, Equation 1 above can be applied to substantially all possible power combinations of the one or more vital sign parameters, the differential parameters, and the one or more vital sign parameters with the differential parameters, subject to the predetermined constraints described above (i.e., the value of $n$ being any integer greater than two, and each of $a, b,$ and $c$ being selected from the group consisting of -2, -1, 0, 1, and 2).

Hypotension profiling parameters 52 include one or more vital sign parameters characterizing vital sign data, the differential parameters, and the combinatorial parameters. Examples of hypotension profiling parameters 52 can include, but are not limited to, various combinations of one or more of the following parameters $v_1, v_2 ... v_{19}$, where:

$v_1$ = CWI, the cardiac work indexed by the body surface area of patient 36;
$v_2$ = MAPavg, the averaged mean arterial pressure;
$v_3$ = ΔMAPavg, the change of the averaged mean arterial pressure MAPavg when compared to an initial state;
$v_4$ = avgSysDec, the averaged pressure at the decay portion of the systolic phase;
$v_5$ = ΔSys, the change of systolic pressure when compared to initial values;
$v_6$ = ppAreaNor, the normalized area under the adjusted arterial pressure waveform;
$v_7$ = biasDia, the bias of the diastolic slope;
$v_8$ = CW, the cardiac work;
$v_9$ = mapDnlocArea, the area under the adjusted arterial pressure waveform, between first instance of MAP and the dicrotic notch;
$v_{10}$ = SWcomb, the stroke work;
$v_{11}$ = ppArea, the area under the adjusted arterial pressure waveform;
$v_{12}$ = decAreaNor, the normalized area of the decay phase;
$v_{13}$ = slopeSys, the slope of the systolic phase;
$v_{14}$ = Cwk, the Winkessel compliance;
$v_{15}$ = sys_rise_area_nor, the normalized area under the systolic rise phase;
$v_{16}$ = pulsepres, the pulse pressure;
$v_{17}$ = avg_sys, the averaged pressure of the systolic phase;
$v_{18}$ = dpdt2, the maximum value of the second order derivative of the adjusted arterial pressure waveform; and
$v_{19}$ = dpdt, the maximum value of the first order derivative of the adjusted arterial pressure waveform.

As such, hypotension prediction software code 48 determines hypotension profiling parameters 52 by identifying one or more vital sign parameters characterizing vital sign data based on the hemodynamic data, obtaining the differential parameters based on one or more vital sign parameters, and generating the combinatorial parameters using one or more of vital sign parameters and the differential parameters.

Hemodynamic monitor 10 generates a set of transformed hypotension profiling parameters (Step 76). For instance, hemodynamic monitor 10 can execute hypotension prediction software code 48 to determine transformed hypotension profiling parameters 53. Each of transformed hypotension profiling parameters 53 can be a function of a corresponding one of hypotension profiling parameters 52 at the standard MAP threshold for hypotension (e.g., 65 mmHg or other defined pressure threshold), a mean of the corresponding one of hypotension profiling parameters 52 at the standard MAP threshold, a standard deviation of the corresponding one of hypotension profiling parameters 52 at the standard MAP threshold, a mean of the corresponding one of hypotension profiling parameters 52 at the adjusted MAP threshold for hypotension, and a standard deviation of the corresponding one of the hypotension profiling parameters 52 at the adjusted MAP threshold for hypotension.

In some examples, hemodynamic monitor 10 executes hypotension prediction software code 48 to determine transformed hypotension profiling parameters 53 according to the following equation:

$$v_{k\theta} = \left(\frac{\sigma_{k\theta}}{\sigma_k}\right) v_k + \left(\mu_{k\theta} - \frac{\sigma_{k\theta}}{\sigma_k}\mu_k\right) \qquad \text{(Equation 2)}$$

where:

$v_{k\theta}$ is one of transformed hypotension profiling parameters 53 associated with a corresponding one of hypotension profiling parameters 52;

$\sigma_{k\theta}$ is the standard deviation of the corresponding one of hypotension profiling parameters 52 at the adjusted MAP threshold for hypotension;

$\sigma_k$ is the standard deviation of the corresponding one of hypotension profiling parameters 52 at the standard MAP threshold for hypotension;

$v_k$ is the corresponding one of hypotension profiling parameters 52 at the standard MAP threshold for hypotension;

$\mu_{k\theta}$ is the mean of the corresponding one of hypotension profiling parameters 52 at the adjusted MAP threshold for hypotension; and

$\mu_k$ is the mean of the corresponding one of hypotension profiling parameters 52 at the standard MAP threshold for hypotension.

Accordingly, hemodynamic monitor 10 can execute hypotension prediction software code 48 to determine, according to Equation 2 above, transformed hypotension profiling parameters 53, which can be represented as the set $v_{1\theta}, v_{2\theta}, ... v_{19\theta}$. Each of transformed hypotension profiling parameters 53 (i.e., each of the set $v_{1\theta}, v_{2\theta}, ... v_{19\theta}$) represents a transformed one of hypotension profiling parameters 52 adapted for the new MAP threshold for hypotension.

A risk score representing a probability of a future hypotension event for patient 36 is determined based on transformed hypotension profiling parameters 53 (Step 78). For example, system processor 40 can execute hypotension prediction software code 48 to cause predictive weighting module 50 to determine the risk score as a weighted combination of transformed hypotension profiling parameters 53. Predictive weighting module 50 can determine the weighted combination of transformed hypotension profiling parameters 53 by applying a plurality of risk coefficients to transformed hypotension profiling parameters 53, which include the vital sign parameters characterizing vital sign data of patient 36, the differential parameters derived from the vital sign parameters, and the combinatorial parameters.

The plurality of risk coefficients applied by predictive weighting module 50 can be determined (e.g., via offline training) with respect to the standard MAP threshold for hypotension. As such, because hypotension prediction software code 48 determines the risk score based on transformed hypotension profiling parameters 53, which are derived from hemodynamic data sensed by hemodynamic sensor attached to patient 36, it should be noted that hypotension prediction software code 48 determines the risk score for patient 36 without direct comparison to hypotension in other patients and without direct reference to a hypotension database that may store information regarding hypotension in patients other than patient 36.

In some examples, predictive weighting module 50 determines the risk score representing the probability of a future hypotension event for patient 36 according to the following equation:

$$R = 1/(1 + e^{-A}) \qquad \text{(Equation 3)}$$

where R is the risk score, and $A$ is expressed as:

$$A = c_0 + c_1 \times v_{1\theta} + c_2 \times v_{2\theta} + c_3 \times v_{3\theta} + c_4 \times v_{4\theta} + c_5 \times v_{5\theta} + c_6 \times v_{6\theta}$$
$$+ c_7 \times v_{7\theta}^2 \times v_{8\theta}^2 \times v_{9\theta}^{-2} + c_8 \times v_{2\theta}^2 \times v_{10\theta} \times v_{11\theta}^{-1} + c_9 \times \Delta\left(v_{12\theta}^2 \times v_{13\theta}^2 \times v_{14\theta}\right)$$
$$+ c_{10} \times \Delta\left(v_{15\theta}^2 \times v_{1\theta} \times v_{16\theta}^{-1}\right) + c_{11} \times \Delta(v_{17\theta}^2 \times v_{18\theta}^2 \times v_{19\theta}^{-2})$$

where:

$v_{1\theta}, v_{2\theta}, ... v_{19\theta}$ are the transformed set of hypotension profiling parameters 53;

$v_{1\theta}$ = the transformed CWI, the cardiac work indexed by the body surface area of patient 36 included in hypotension

profiling parameters;

$v_{2\theta}$ = the transformed MAPavg, the averaged mean arterial pressure;

$v_{3\theta}$ = the transformed $\Delta$MAPavg, the change of the averaged mean arterial pressure MAPavg when compared to an initial state;

$v_{4\theta}$ = the transformed avgSysDec, the averaged pressure at the decay portion of the systolic phase;

$v_{5\theta}$ = the transformed $\Delta$Sys, the change of systolic pressure when compared to initial values;

$v_{6\theta}$ = the transformed ppAreaNor, the normalized area under the adjusted arterial pressure waveform;

$v_{7\theta}$ = the transformed biasDia, the bias of the diastolic slope;

$v_{8\theta}$ = the transformed CW, the cardiac work;

$v_{9\theta}$ = the transformed mapDnlocArea, the area under the adjusted arterial pressure waveform, between first instance of MAP and the dicrotic notch;

$v_{10\theta}$ = the transformed SWcomb, the stroke work;

$v_{11\theta}$ = the transformed ppArea, the area under the adjusted arterial pressure waveform;

$v_{12\theta}$ = the transformed decAreaNor, the normalized area of the decay phase;

$v_{13\theta}$ = the transformed slopeSys, the slope of the systolic phase;

$v_{14\theta}$ = the transformed Cwk, the Windkessel compliance;

$v_{15\theta}$ = the transformed sys_rise_area_nor, the normalized area under the systolic rise phase;

$v_{16\theta}$ = the transformed pulsepres, the pulse pressure;

$v_{17\theta}$ = the transformed avg_sys, the averaged pressure of the systolic phase;

$v_{18\theta}$ = the transformed dpdt2, the maximum value of the second order derivative of the adjusted arterial pressure waveform;

$v_{19\theta}$ = the transformed dpdt, the maximum value of the first order derivative of the adjusted arterial pressure waveform; and

$c_0, c_1, ..., c_{11}$ are the risk coefficients determined with respect to the standard MAP threshold for hypotension.

In some examples, the risk score R can be expressed as a fraction, as represented by Equation 3 above. In other examples, the risk score can be converted to a percentage risk score between zero percent and one hundred percent.

Hemodynamic monitor 10 invokes a sensory alarm in response to the risk score satisfying a predetermined risk criterion (Step 80). For instance, hypotension prediction software code 48 can invoke sensory alarm 58 of user interface 54 in response to determining that the risk score $R$ determined according to Equation 3 above satisfies a predetermined risk criterion. In some examples, the output of hypotension prediction software code 48 can be processed using DAC 46 to convert digital signals into analog signals for presentation via user interface 54 at display 12.

The predetermined risk criterion can be based on the value of the risk score, on the trend of the risk score over a time interval, or both. For instance, where the risk score is expressed as a percentage between zero and one hundred, hypotension prediction software code 48 can invoke sensory alarm 58 (e.g., immediately) in response to determining that the risk score exceeds a first predetermined threshold, such as eighty-five percent. In some examples, hypotension prediction software code 48 can invoke sensory alarm 58 in response to determining that the risk score satisfies a second predetermined threshold over the entirety of a first predetermined time period. In such examples, the second predetermined threshold can be lower than the first predetermined threshold.

As such, hypotension prediction software code 48 can invoke sensory alarm 58, e.g., immediately, in response to determining that the risk score exceeds the first predetermined threshold (e.g., eighty-five percent). Hypotension prediction software code 48 can also invoke sensory alarm 58 in response to determining that the risk score exceeds a second predetermined threshold (e.g., eighty percent) that is less than the first predetermined threshold for the first predetermined time period (e.g., ten to thirty seconds) during which the risk score is continuously greater than the second predetermined threshold (eighty percent) and less than the first predetermined threshold (e.g., eighty five percent). In certain examples, hypotension prediction software code 48 can invoke sensory alarm 58 in response to determining that the risk score is greater than a third predetermined threshold that is less than the second predetermined threshold for a second predetermined time period (e.g., one or more minutes). In yet other examples, hypotension prediction software code 48 can invoke sensory alarm 58 in response to determining that the risk score exceeds a fourth predetermined threshold (e.g., seventy-five percent) a threshold number of times (e.g., two times, three times, or other numbers of times) over a third predetermined time period (e.g., one minute, two minutes, or other time periods).

Although not illustrated in the example operations of FIG. 6, in some examples, hemodynamic monitor 10 can identify, using hypotension prediction software code 48, a most probable cause of a predicted future hypotension event of patient 36. For example, based on identified indicia, hypotension prediction software code 48 can identify poor vascular tone, low blood volume, reduced cardiac contractility, or other most probable causes of a predicted future hypotension event of patient 36.

In some examples, hemodynamic monitor 10 can recommend a medical intervention for preventing the predicted future hypotension event of patient 36, such as by identifying a recommended medical intervention corresponding to the identified most probable cause of the predicted future hypotension event for patient 36. For instance, with respect to a most probable cause of poor vascular tone, hemodynamic monitor 10 can recommend a medical intervention of administration of a vasoconstrictor. With respect to a most probable cause of low blood volume, for example, hemodynamic monitor 10 can recommend a medical intervention of administration of saline or whole blood.

Accordingly, hemodynamic monitor 10 implementing techniques of this disclosure provides a risk score predictive of a future hypotension event for patient 36, thereby enabling timely and effective intervention to prevent the hypotension event prior to patient 36 entering a hypotensive state. Moreover, by enabling adjustment to a defined hypotension threshold without requiring retraining of the predictive risk model, the techniques described herein increase usability of hemodynamic monitoring system 32 to accommodate, e.g., the training and experience of medical personnel.

While the invention has been described with reference to an exemplary embodiment(s), it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment(s) disclosed, but that the invention will include all embodiments falling within the scope of the appended claims.

## Claims

1. A method for monitoring of arterial pressure of a patient and providing a warning to medical personnel of a predicted future hypotension event of the patient, the method comprising:

    receiving, by a hemodynamic monitor, an adjusted mean arterial pressure (MAP) threshold for hypotension;
    receiving, by the hemodynamic monitor, sensed hemodynamic data representative of an arterial pressure waveform of the patient;
    performing, by the hemodynamic monitor, waveform analysis of the hemodynamic data to determine a plurality of hypotension profiling parameters predictive of a future hypotension event for the patient;
    generating, by the hemodynamic monitor, a set of transformed hypotension profiling parameters, each transformed hypotension profiling parameter being a function of a corresponding one of the plurality of hypotension profiling parameters at a standard mean arterial pressure (MAP) threshold for hypotension, a mean of the corresponding one of the plurality of hypotension profiling parameters at the standard MAP threshold for hypotension, a standard deviation of the corresponding one of the plurality of hypotension profiling parameters at the standard MAP threshold, a mean of the corresponding one of the plurality of hypotension profiling parameters at the adjusted MAP threshold for hypotension, and a standard deviation of the corresponding one of the plurality of hypotension profiling parameters at the adjusted MAP threshold for hypotension;
    determining, by the hemodynamic monitor based on the set of transformed hypotension profiling parameters, a risk score representing a probability of the future hypotension event for the patient; and
    invoking, by the hemodynamic monitor, a sensory alarm to produce a sensory signal in response to the risk score satisfying a predetermined risk criterion.

2. The method of claim 1,

    wherein generating the set of transformed hypotension profiling parameters comprises transforming each of the plurality of hypotension profiling parameters according to the following equation:

$$ v_{k\theta} = \left( \frac{\sigma_{k\theta}}{\sigma_k} \right) v_k + \left( \mu_{k\theta} - \frac{\sigma_{k\theta}}{\sigma_k} \mu_k \right) $$

    wherein $v_{k\theta}$ is one of the set of transformed set of hypotension profiling parameters associated with the corresponding one of the plurality of hypotension profiling parameters;
    wherein $\sigma_{k\theta}$ is the standard deviation of the corresponding one of the plurality of hypotension profiling parameters at the adjusted MAP threshold for hypotension;
    wherein $\sigma_k$ is the standard deviation of the corresponding one of the plurality of hypotension profiling parameters at the standard MAP threshold for hypotension;
    wherein $v_k$ is the corresponding one of the plurality of hypotension profiling parameters at the standard MAP threshold for hypotension;

wherein $\mu_{k\theta}$ is the mean of the corresponding one of the plurality of hypotension profiling parameters at the adjusted MAP threshold for hypotension; and

wherein $\mu_k$ is the mean of the corresponding one of the plurality of hypotension profiling parameters at the standard MAP threshold for hypotension.

3. The method of claim 1 or claim 2,
wherein determining the risk score representing the probability of the future hypotension event for the patient comprises applying a plurality of risk coefficients to the set of transformed hypotension profiling parameters to determine the risk score.

4. The method of claim 3,
wherein the plurality of risk coefficients are determined based on the standard MAP threshold.

5. The method of any preceding claim,
wherein performing the waveform analysis of the hemodynamic data to determine the plurality of hypotension profiling parameters predictive of the future hypotension event for the patient comprises:

performing the waveform analysis of the hemodynamic data to obtain vital sign parameters from the hemodynamic data;
deriving differential parameters based on one or more of the vital sign parameters; and
generating combinatorial parameters using one or more of the vital sign parameters and/or one or more of the differential parameters;
wherein the plurality of hypotension profiling parameters include one or more of the vital sign parameters, the differential parameters, and the combinatorial parameters.

6. The method of claim 5,
wherein the vital sign parameters include one or more of stroke volume, heart rate, respiration, and cardiac contractibility.

7. The method of claim 5 or claim 6,
wherein deriving the differential parameters based on one or more of the vital sign parameters comprises deriving the differential parameters to represent variations in the one or more of the vital sign parameters with respect to time, with respect to frequency, or with respect to other vital sign parameters.

8. The method of any of claims 5 to 7,
wherein generating the combinatorial parameters comprises generating the combinatorial parameters as a combination of vital sign parameters, a combination of differential parameters, or a combination of at least one vital sign parameter and at least one differential parameter.

9. A system for monitoring of arterial pressure of a patient and providing a warning to medical personnel of a predicted future hypotension event of the patient, the system comprising:

a hemodynamic sensor that produces hemodynamic data representative of an arterial pressure waveform of the patient;
a system memory that stores hypotension prediction software code including a predictive weighting module;
a user interface that includes a sensory alarm that provides a sensory signal to warn the medical personnel of the predicted future hypotension event prior to the patient entering a hypotensive state, and control elements configured to enable user input of an adjusted MAP threshold for hypotension; and
a hardware processor that is configured to execute the hypotension prediction software code to:

perform waveform analysis of the hemodynamic data to determine a plurality of hypotension profiling parameters predictive of a future hypotension event for the patient;
generate a set of transformed hypotension profiling parameters, each transformed hypotension profiling parameter being a function of a corresponding one of the plurality of hypotension profiling parameters at a standard mean arterial pressure (MAP) threshold for hypotension, a mean of the corresponding one of the plurality of hypotension profiling parameters at the standard MAP threshold for hypotension, a standard deviation of the corresponding one of the plurality of hypotension profiling parameters at the standard MAP threshold, a mean of the corresponding one of the plurality of hypotension profiling parameters at the adjusted MAP threshold for hypotension, and a standard deviation of the corresponding one of the plurality of hypotension profiling parameters at the adjusted MAP threshold for hypotension;
determine, based on the set of transformed hypotension profiling parameters, a risk score representing a probability of the future hypotension event for the patient; and
invoke the sensory alarm of the user interface in response to the risk score satisfying a predetermined risk criterion.

**10.** The system of claim 9,

wherein the hardware processor is configured to generate the set of transformed hypotension profiling parameters according to the following equation:

$$ v_{k\theta} = \left( \frac{\sigma_{k\theta}}{\sigma_k} \right) v_k + \left( \mu_{k\theta} - \frac{\sigma_{k\theta}}{\sigma_k} \mu_k \right) $$

wherein $v_{k\theta}$ is one of the set of transformed set of hypotension profiling parameters associated with the corresponding one of the plurality of hypotension profiling parameters;
wherein $\sigma_{k\theta}$ is the standard deviation of the corresponding one of the plurality of hypotension profiling parameters at the adjusted MAP threshold for hypotension;
wherein $\sigma_k$ is the standard deviation of the corresponding one of the plurality of hypotension profiling parameters at the standard MAP threshold for hypotension;
wherein $v_k$ is the corresponding one of the plurality of hypotension profiling parameters at the standard MAP threshold for hypotension;
wherein $\mu_{k\theta}$ is the mean of the corresponding one of the plurality of hypotension profiling parameters at the adjusted MAP threshold for hypotension; and
wherein $\mu_k$ is the mean of the corresponding one of the plurality of hypotension profiling parameters at the standard MAP threshold for hypotension.

**11.** The system of claim 9 or claim 10,

wherein the hardware processor is configured to execute the hypotension prediction software code to determine the risk score representing the probability of the future hypotension event for the patient by applying, using the predictive weighting module, a plurality of risk coefficients to the plurality of hypotension profiling parameters to determine the risk score,
optionally wherein the plurality of risk coefficients are determined based on the standard MAP threshold.

**12.** The system of any of claims 9 to 11,
wherein the hardware processor is configured to execute the hypotension prediction software code to determine the plurality of hypotension profiling parameters predictive of the future hypotension event for the patient by executing the hypotension prediction software code to:

perform the waveform analysis of the hemodynamic data to obtain vital sign parameters from the adjusted hemodynamic data;
derive differential parameters based on one or more of the vital sign parameters; and
generate combinatorial parameters using one or more of the vital sign parameters and/or one or more of the differential parameters;
wherein the plurality of hypotension profiling parameters include one or more of the vital sign parameters, the differential parameters, and the combinatorial parameters.

**13.** The system of claim 12,
wherein the vital sign parameters include one or more of stroke volume, heart rate, respiration, and cardiac contractibility.

**14.** The system of claim 12 or claim 13,
wherein the hardware processor is configured to execute the hypotension prediction software code to:

a) derive the differential parameters based on one or more of the vital sign parameters by deriving the differential parameters to represent variations in the one or more of the vital sign parameters with respect to time, with respect to frequency, or with respect to other vital sign parameters; or

b) generate the combinatorial parameters by generating the combinatorial parameters as a combination of vital sign parameters, a combination of differential parameters, or a combination of at least one vital sign parameter and at least one differential parameter.

15. The system of any of claims 9 to 14,
   wherein the hemodynamic sensor:

a) is a noninvasive hemodynamic sensor that is attachable to an extremity of the patient;

b) is a minimally invasive arterial catheter based hemodynamic sensor; or

c) produces the hemodynamic data as an analog hemodynamic sensor signal representative of the arterial pressure waveform of the patient, optionally further comprising:

an analog-to-digital converter that converts the analog hemodynamic sensor signal to digital hemodynamic data representative of the arterial pressure waveform of the patient.

**Patentansprüche**

1. Verfahren zum Überwachen von arteriellem Druck eines Patienten und zum Bereitstellen einer Warnung an medizinisches Personal in Bezug auf ein vorhergesagtes zukünftiges Hypotonieereignis des Patienten, das Verfahren umfassend:

Empfangen, durch einen Hämodynamikmonitor, eines angepassten Schwellenwerts für den mittleren arteriellen Blutdruck (mean arterial pressure - MAP) in Bezug auf Hypotonie;

Empfangen, durch den Hämodynamikmonitor, von erfassten Hämodynamikdaten, die einer Wellenform des arteriellen Drucks des Patienten entsprechen;

Durchführen, durch den Hämodynamikmonitor, einer Wellenformanalyse der Hämodynamikdaten, um eine Vielzahl von Hypotonieprofilierungsparametern zu bestimmen, die ein zukünftiges Hypotonieereignis für den Patienten vorhersagen;

Erzeugen, durch den Hämodynamikmonitor, eines Satzes von umgewandelten Hypotonieprofilierungsparametern, wobei jeder Hypotonieprofilierungsparameter eine Funktion eines entsprechenden einen der Vielzahl von Hypotonieprofilierungsparametern an einem Standardschwellenwert für mittleren arteriellen Druck (MAP) für Hypotonie ist, eines Mittelwerts des entsprechenden einen der Vielzahl von Hypotonieprofilierungsparametern am Standard-MAP-Schwellenwert für Hypotonie, einer Standardabweichung des entsprechenden einen der Vielzahl von Hypotonieprofilierungsparametern am Standard-MAP-Schwellenwert, eines Mittelwerts des entsprechenden einen der Vielzahl von Hypotonieprofilierungsparametern am angepassten MAP-Schwellenwert für Hypotonie sowie einer Standardabweichung des entsprechenden einen der Vielzahl von Hypotonieprofilierungsparametern am angepassten MAP-Schwellenwert für Hypotonie;

Bestimmen, durch den Hämodynamikmonitor, auf Grundlage des Satzes von umgewandelten Hypotonieprofilierungsparametern, einer Risikobewertung, die einer Wahrscheinlichkeit des zukünftigen Hypotonieereignisses für den Patienten entspricht; und

Aufrufen, durch den Hämodynamikmonitor, eines Sensoralarms, um ein Sensorsignal als Reaktion darauf zu erzeugen, dass die Risikobewertung ein vorbestimmtes Risikokriterium erfüllt.

2. Verfahren nach Anspruch 1,

wobei das Erzeugen des Satzes von umgewandelten Hypotonieprofilierungsparametern das Umwandeln von dem der Vielzahl von Hypotonieprofilierungsparametern gemäß der folgenden Gleichung umfasst:

$$v_{k\theta} = \left(\frac{\sigma_{k\theta}}{\sigma_k}\right) v_k + \left(\mu_{k\theta} - \frac{\sigma_{k\theta}}{\sigma_k} \mu_k\right)$$

wobei $v_{k\theta}$ einer aus dem Satz des umgewandelten Satzes von Hypotonieprofilierungsparametern ist, die mit dem entsprechenden einen der Vielzahl von Hypotonieprofilierungsparametern verknüpft sind;

wobei $\sigma_{k\theta}$ die Standardabweichung des entsprechenden einen der Vielzahl von Hypotonieprofilierungsparameter am angepassten MAP-Schwellenwert für Hypotonie ist;

wobei $\sigma_k$ die Standardabweichung des entsprechenden einen der Vielzahl von Hypotonieprofilierungsparametern am Standard-MAP-Schwellenwert für Hypotonie ist;

wobei $v_k$ der entsprechende eine der Vielzahl von Hypotonieprofilierungsparametern am Standard-MAP-Schwellenwert für Hypotonie ist;

wobei $\mu_{k\theta}$ der Mittelwert des entsprechenden einen der Vielzahl von Hypotonieprofilierungsparametern am angepassten MAP-Schwellenwert für Hypotonie ist; und wobei $\mu_k$ der Mittelwert des entsprechenden einen der Vielzahl von Hypotonieprofilierungsparametern am Standard-MAP-Schwellenwert für Hypotonie ist.

3. Verfahren nach Anspruch 1 oder 2,
wobei das Bestimmen der Risikobewertung, die der Wahrscheinlichkeit des zukünftigen Hypotonieereignisses für den Patienten entspricht, das Anwenden einer Vielzahl von Risikokoeffizenten auf den Satz von umgewandelten Hypotonieprofilierungsparametern umfasst, um die Risikobewertung zu bestimmen.

4. Verfahren nach Anspruch 3,
wobei die Vielzahl von Risikokoeffizenten auf der Grundlage des Standard-MAP-Schwellenwerts bestimmt wird.

5. Verfahren nach einem der vorhergehenden Ansprüche, wobei das Durchführen der Wellenformanalyse der Hämodynamikdaten, um die Vielzahl von Hypotonieprofilierungsparametern zu bestimmen, die das zukünftige Hypotonieereignis für den Patienten vorhersagen, umfasst:

Durchführen der Wellenformanalyse der Hämodynamikdaten, um Vitalzeichenparameter aus den Hämodynamikdaten zu erhalten;
Ableiten von Differentialparametern auf der Grundlage von einem oder mehreren der Vitalzeichenparameter; und
Erzeugen von kombinatorischen Parametern unter Einsatz von einem oder mehreren der Vitalzeichenparameter und/oder einem oder mehreren der Differentialparameter; wobei die Vielzahl von Hypotonieprofilierungsparametern einen oder mehrere der Vitalzeichenparameter, der Differentialparameter und der kombinatorischen Parameter einschließt.

6. Verfahren nach Anspruch 5,
wobei die Vitalzeichenparameter eines oder mehrere von Schlagvolumen, Herzfrequenz, Atmung und Herzkontraktibilität einschließen.

7. Verfahren nach Anspruch 5 oder 6,
wobei das Ableiten der Differentialparameter auf der Grundlage von einem oder mehreren der Vitalzeichenparameter das Ableiten der Differentialparameter derart umfasst, dass sie Variationen an einem oder mehreren der Vitalzeichenparameter darstellen, in Bezug auf Zeit, in Bezug auf Frequenz oder in Bezug auf andere Vitalzeichenparameter.

8. Verfahren nach einem der Ansprüche 5 bis 7,
wobei das Erzeugen der kombinatorischen Parameter das Erzeugen der kombinatorischen Parameter als eine Kombination aus Vitalzeichenparametern oder als eine Kombination aus Differentialparametern oder als eine Kombination aus mindestens einem Vitalzeichenparameter und mindestens einem Differentialparameter umfasst.

9. System zum Überwachen von arteriellem Druck eines Patienten und zum Bereitstellen einer Warnung an medizinisches Personal in Bezug auf ein vorhergesagtes zukünftiges Hypotonieereignis des Patienten, das System umfassend:

einen Hämodynamiksensor, der Hämodynamikdaten erzeugt, die einer Wellenform des arteriellen Drucks des Patienten entsprechen;
einen Systemspeicher, in dem Hypotonievorhersagesoftwarecode gespeichert ist, einschließlich eines Vorhersagegewichtungsmoduls;
eine Benutzerschnittstelle, einschließlich eines Sensoralarms, welcher ein Sensorsignal bereitstellt, um das medizinische Personal vor dem vorhergesagten zukünftigen Hypotonieereignis zu warnen, bevor der Patient in einen Hypotoniestatus übergeht, und Kontrollelemente, dazu konfiguriert, eine Benutzereingabe eines angepassten MAP-Schwellenwerts für Hypotonie zu ermöglichen; und
einen Hardwareprozessor, dazu konfiguriert, den Hypotonievorhersagesoftwarecode auszuführen, zum:

Durchführen einer Wellenformanalyse der Hämodynamikdaten, um eine Vielzahl von Hypotonieprofilierungsparametern zu bestimmen, die ein zukünftiges Hypotonieereignis für den Patienten vorhersagen;

Erzeugen eines Satzes von umgewandelten Hypotonieprofilierungsparametern, wobei jeder Hypotonieprofilierungsparameter eine Funktion eines entsprechenden einen der Vielzahl von Hypotonieprofilierungsparametern an einem Standardschwellenwert für mittleren arteriellen Druck (MAP) für Hypotonie ist, eines Mittelwerts des entsprechenden einen der Vielzahl von Hypotonieprofilierungsparametern am Standard-MAP-Schwellenwert für Hypotonie, einer Standardabweichung des entsprechenden einen der Vielzahl von Hypotonieprofilierungsparametern am Standard-MAP-Schwellenwert, eines Mittelwerts des entsprechenden einen der Vielzahl von Hypotonieprofilierungsparametern am angepassten MAP-Schwellenwert für Hypotonie sowie einer Standardabweichung des entsprechenden einen der Vielzahl von Hypotonieprofilierungsparametern am angepassten MAP-Schwellenwert für Hypotonie;

Bestimmen, auf Grundlage des Satzes von umgewandelten Hypotonieprofilierungsparametern, einer Risikobewertung, die einer Wahrscheinlichkeit des zukünftigen Hypotonieereignisses für den Patienten entspricht; und

Aufrufen des Sensoralarms der Benutzerschnittstelle als Reaktion darauf, dass die Risikobewertung ein vorbestimmtes Risikokriterium erfüllt.

**10.** System nach Anspruch 9,

wobei der Hardwareprozessor dazu konfiguriert ist, den Satz von umgewandelten Hypotonieprofilierungsparametern gemäß der folgenden Gleichung zu erzeugen:

$$v_{k\theta} = \left(\frac{\sigma_{k\theta}}{\sigma_k}\right) v_k + \left(\mu_{k\theta} - \frac{\sigma_{k\theta}}{\sigma_k}\mu_k\right)$$

wobei $v_{k\theta}$ einer aus dem Satz des umgewandelten Satzes von Hypotonieprofilierungsparametern ist, die mit dem entsprechenden einen der Vielzahl von Hypotonieprofilierungsparametern verknüpft sind;

wobei $\sigma_{k\theta}$ die Standardabweichung des entsprechenden einen der Vielzahl von Hypotonieprofilierungsparameter am angepassten MAP-Schwellenwert für Hypotonie ist;

wobei $\sigma_k$ die Standardabweichung des entsprechenden einen der Vielzahl von Hypotonieprofilierungsparametern am Standard-MAP-Schwellenwert für Hypotonie ist;

wobei $v_k$ der entsprechende eine der Vielzahl von Hypotonieprofilierungsparametern am Standard-MAP-Schwellenwert für Hypotonie ist;

wobei $\mu_{k\theta}$ der Mittelwert des entsprechenden einen der Vielzahl von Hypotonieprofilierungsparametern am angepassten MAP-Schwellenwert für Hypotonie ist; und wobei $\mu_k$ der Mittelwert des entsprechenden einen der Vielzahl von Hypotonieprofilierungsparametern am Standard-MAP-Schwellenwert für Hypotonie ist.

**11.** System nach Anspruch 9 oder 10,

wobei der Hardwareprozessor dazu konfiguriert ist, den Hypotonievorhersagesoftwarecode auszuführen, um die Risikobewertung zu bestimmen, die der Wahrscheinlichkeit des zukünftigen Hypotonieereignisses für den Patienten entspricht, indem er unter Einsatz des Vorhersagegewichtungsmoduls eine Vielzahl von Risikokoeffizienten auf die Vielzahl von Hypotonieprofilierungsparametern anwendet, um die Risikobewertung zu bestimmen,

optional wobei die Vielzahl von Risikokoeffizenten auf der Grundlage des Standard-MAP-Schwellenwerts bestimmt wird.

**12.** System nach einem der Ansprüche 9 bis 11,
wobei der Hardwareprozessor dazu konfiguriert ist, den Hypotonievorhersagesoftwarecode auszuführen, um die Vielzahl von Hypotonieprofilierungsparametern zu bestimmen, die das zukünftige Hypotonieereignis für den Patienten vorhersagen, indem er den Hypotonievorhersagesoftwarecode ausführt zum:

Durchführen der Wellenformanalyse der Hämodynamikdaten, um Vitalzeichenparameter aus den Hämodynamikdaten zu erhalten;

Ableiten von Differentialparametern auf der Grundlage von einem oder mehreren der Vitalzeichenparameter; und

Erzeugen von kombinatorischen Parametern unter Einsatz von einem oder mehreren der Vitalzeichenparameter und/oder einem oder mehreren der Differentialparameter; wobei die Vielzahl von Hypotonieprofilierungsparametern einen oder mehrere der Vitalzeichenparameter, der Differentialparameter und der kombinatorischen Parameter einschließt.

**13.** System nach Anspruch 12,
wobei die Vitalzeichenparameter eines oder mehrere von Schlagvolumen, Herzfrequenz, Atmung und Herzkontraktibilität einschließen.

**14.** System nach Anspruch 12 oder 13,
wobei der Hardwareprozessor, dazu konfiguriert ist, den Hypotonievorhersagesoftwarecode auszuführen, zum:

a) Ableiten der Differentialparameter auf der Grundlage von einem oder mehreren der Vitalzeichenparameter durch Ableiten der Differentialparameter derart, dass sie Variationen an einem oder mehreren der Vitalzeichenparameter darstellen, in Bezug auf Zeit, in Bezug auf Frequenz oder in Bezug auf andere Vitalzeichenparameter; oder
b) Erzeugen der kombinatorischen Parameter durch Erzeugen der kombinatorischen Parameter als eine Kombination aus Vitalzeichenparametern oder als eine Kombination aus Differentialparametern oder als eine Kombination aus mindestens einem Vitalzeichenparameter und mindestens einem Differentialparameter.

**15.** System nach einem der Ansprüche 9 bis 14,
wobei der Hämodynamiksensor:

a) ein nichtinvasiver Hämodynamiksensor ist, der an einer Extremität
des Patienten befestigt werden kann;
b) ein minimalinvasiver Hämodynamiksensor auf Arterienkatheterbasis ist; oder
c) die Hämodynamikdaten als analoges Hämodynamiksensorsignal erzeugt, das der Wellenform des arteriellen Drucks des Patienten entspricht, optional ferner umfassend:
einen Analog-Digital-Wandler, der das analoge Hämodynamiksensorsignal in digitale Hämodynamikdaten umwandelt, die der Wellenform des arteriellen Drucks des Patienten entsprechen.

**Revendications**

**1.** Procédé de surveillance de la pression artérielle d'un patient et de fourniture d'un avertissement au personnel médical d'un événement d'hypotension futur prédit du patient, le procédé comprenant :

la réception, par un moniteur hémodynamique, d'un seuil de pression artérielle moyenne (MAP) ajusté pour l'hypotension ;
la réception, par le moniteur hémodynamique, de données hémodynamiques détectées représentatives d'une forme d'onde de pression artérielle du patient ;
la réalisation, par le moniteur hémodynamique, d'une analyse de la forme d'onde des données hémodynamiques afin de déterminer une pluralité de paramètres de profilage d'hypotension prédictifs d'un événement d'hypotension futur chez le patient ;
la génération, par le moniteur hémodynamique, d'un ensemble de paramètres de profilage d'hypotension transformés, chaque paramètre de profilage d'hypotension transformé étant une fonction d'un paramètre correspondant de la pluralité de paramètres de profilage d'hypotension à un seuil de pression artérielle moyenne (MAP) standard pour l'hypotension, une moyenne du paramètre correspondant de la pluralité de paramètres de profilage d'hypotension au seuil de MAP standard pour l'hypotension, un écart-type du paramètre correspondant de la pluralité de paramètres de profilage d'hypotension à un seuil de MAP standard pour l'hypotension, une moyenne du paramètre correspondant de la pluralité de paramètres de profilage d'hypotension au seuil de MAP ajusté pour l'hypotension, et un écart-type du paramètre correspondant de la pluralité de paramètres de profilage d'hypotension au seuil de MAP ajusté pour l'hypotension ;
la détermination, par le moniteur hémodynamique sur la base de l'ensemble de paramètres de profilage d'hypotension transformés, d'un score de risque représentant une probabilité de l'événement d'hypotension futur chez le patient ; et
le déclenchement, par le moniteur hémodynamique, d'une alarme sensorielle pour produire un signal sensoriel en réponse au score de risque satisfaisant un critère de risque prédéterminé.

**2.** Procédé selon la revendication 1,

la génération de l'ensemble de paramètres de profilage d'hypotension transformés comprenant la transformation de chacun de la pluralité de paramètres de profilage d'hypotension selon l'équation suivante :

$$v_{k\theta} = \left(\frac{\sigma_{k\theta}}{\sigma_k}\right) v_k + \left(\mu_{k\theta} - \frac{\sigma_{k\theta}}{\sigma_k}\mu_k\right)$$

$v_{k\theta}$ étant l'un des ensembles de paramètres de profilage d'hypotension transformés associés à l'ensemble correspondant de la pluralité de paramètres de profilage d'hypotension ;

$\sigma_{k\theta}$ étant l'écart-type du paramètre correspondant de la pluralité de paramètres de profilage d'hypotension au seuil de MAP ajusté pour l'hypotension ;

$\sigma_k$ étant l'écart-type du paramètre correspondant de la pluralité de paramètres de profilage d'hypotension au seuil de MAP standard pour l'hypotension ;

$v_k$ étant le paramètre correspondant de la pluralité de paramètres de profilage d'hypotension au seuil de MAP standard pour l'hypotension ;

$\mu_{k\theta}$ étant la moyenne du paramètre correspondant de la pluralité de paramètres de profilage d'hypotension au seuil de MAP ajusté pour l'hypotension ; et

$\mu_k$ étant la moyenne du paramètre correspondant de la pluralité de paramètres de profilage d'hypotension au seuil de MAP standard pour l'hypotension.

3. Procédé selon la revendication 1 ou selon la revendication 2,
la détermination du score de risque représentant la probabilité de l'événement d'hypotension futur chez le patient comprenant l'application d'une pluralité de coefficients de risque à l'ensemble de paramètres de profilage d'hypotension transformés pour déterminer le score de risque.

4. Procédé selon la revendication 3,
la pluralité de coefficients de risque étant déterminée sur la base du seuil de MAP standard.

5. Procédé selon n'importe quelle revendication précédente,
la réalisation de l'analyse de la forme d'onde des données hémodynamiques pour déterminer la pluralité de paramètres de profilage d'hypotension prédisant l'événement d'hypotension futur chez le patient comprenant :

la réalisation de l'analyse de la forme d'onde des données hémodynamiques pour obtenir des paramètres de signes vitaux à partir des données hémodynamiques ;
la dérivation de paramètres différentiels basés sur un ou plusieurs des paramètres de signes vitaux ; et
la génération de paramètres combinatoires en utilisant un ou plusieurs des paramètres de signes vitaux et/ou un ou plusieurs des paramètres différentiels ;
la pluralité de paramètres de profilage d'hypotension comprenant un ou plusieurs des paramètres de signes vitaux, des paramètres différentiels et des paramètres combinatoires.

6. Procédé selon la revendication 5,
les paramètres de signes vitaux comprenant un ou plusieurs des paramètres suivants : volume systolique, fréquence cardiaque, respiration et contractilité cardiaque.

7. Procédé selon la revendication 5 ou selon la revendication 6,
la dérivation des paramètres différentiels sur la base d'un ou plusieurs paramètres de signes vitaux comprenant la dérivation des paramètres différentiels pour représenter les variations d'un ou plusieurs paramètres de signes vitaux par rapport au temps, à la fréquence ou à d'autres paramètres de signes vitaux.

8. Procédé selon l'une quelconque des revendications 5 à 7,
la génération des paramètres combinatoires comprenant la génération des paramètres combinatoires comme une combinaison de paramètres de signes vitaux, une combinaison de paramètres différentiels, ou une combinaison d'au moins un paramètre de signe vital et d'au moins un paramètre différentiel.

9. Système de surveillance de la pression artérielle d'un patient et de fourniture d'un avertissement au personnel médical d'un événement d'hypotension futur prédit du patient, le système comprenant :

un capteur hémodynamique qui produit des données hémodynamiques représentatives d'une forme d'onde de pression artérielle du patient ;
une mémoire système qui stocke le code logiciel de prédiction d'hypotension, comprenant un module de pondération prédictive ;

une interface utilisateur qui comprend une alarme sensorielle qui fournit un signal sensoriel pour avertir le personnel médical de l'événement d'hypotension futur prédit avant que le patient n'entre dans un état d'hypotension, et des éléments de commande configurés pour permettre à l'utilisateur de saisir un seuil de MAP ajusté pour l'hypotension ; et

un processeur matériel configuré pour exécuter le code logiciel de prédiction d'hypotension pour :

réaliser une analyse de la forme d'onde des données hémodynamiques pour déterminer une pluralité de paramètres de profilage d'hypotension prédictifs d'un événement d'hypotension futur chez le patient ;

générer un ensemble de paramètres de profilage d'hypotension transformés, chaque paramètre de profilage d'hypotension transformé étant une fonction d'un paramètre correspondant de la pluralité de paramètres de profilage d'hypotension à un seuil de pression artérielle moyenne (MAP) standard pour l'hypotension, une moyenne du paramètre correspondant de la pluralité de paramètres de profilage d'hypotension au seuil de MAP standard pour l'hypotension, un écart-type du paramètre correspondant de la pluralité de paramètres de profilage d'hypotension au seuil de MAP standard pour l'hypotension, une moyenne du paramètre correspondant de la pluralité de paramètres de profilage d'hypotension au seuil de MAP ajusté pour l'hypotension, et un écart-type du paramètre correspondant de la pluralité de paramètres de profilage d'hypotension au seuil de MAP ajusté pour l'hypotension ;

déterminer, sur la base de l'ensemble de paramètres de profilage d'hypotension transformés, un score de risque représentant une probabilité de l'événement d'hypotension futur chez le patient ; et

déclencher l'alarme sensorielle de l'interface utilisateur si le score de risque satisfait à un critère de risque prédéterminé.

10. Système selon la revendication 9,

le processeur matériel étant configuré pour générer l'ensemble de paramètres de profilage d'hypotension transformés selon l'équation suivante :

$$v_{k\theta} = \left(\frac{\sigma_{k\theta}}{\sigma_k}\right) v_k + \left(\mu_{k\theta} - \frac{\sigma_{k\theta}}{\sigma_k}\mu_k\right)$$

$v_{k\theta}$ étant l'un des ensembles de paramètres de profilage d'hypotension transformés associés à l'ensemble correspondant de la pluralité de paramètres de profilage d'hypotension ;

$\sigma_{k\theta}$ étant l'écart-type du paramètre correspondant de la pluralité de paramètres de profilage d'hypotension au seuil de MAP ajusté pour l'hypotension ;

$\sigma_k$ étant l'écart-type du paramètre correspondant de la pluralité de paramètres de profilage d'hypotension au seuil de MAP standard pour l'hypotension ;

$v_k$ étant le paramètre correspondant de la pluralité de paramètres de profilage d'hypotension au seuil de MAP standard pour l'hypotension ;

$\mu_{k\theta}$ étant la moyenne du paramètre correspondant de la pluralité de paramètres de profilage d'hypotension au seuil de MAP ajusté pour l'hypotension ; et

$\mu_k$ étant la moyenne du paramètre correspondant de la pluralité de paramètres de profilage d'hypotension au seuil de MAP standard pour l'hypotension.

11. Système selon la revendication 9 ou selon la revendication 10,

le processeur matériel étant configuré pour exécuter le code logiciel de prédiction d'hypotension pour déterminer le score de risque représentant la probabilité de l'événement d'hypotension futur chez le patient en appliquant, à l'aide du module de pondération prédictive, une pluralité de coefficients de risque à la pluralité de paramètres de profilage d'hypotension pour déterminer le score de risque,

éventuellement, la pluralité de coefficients de risque étant déterminée sur la base du seuil de MAP standard.

12. Système selon l'une quelconque des revendications 9 à 11,
le processeur matériel étant configuré pour exécuter le code logiciel de prédiction d'hypotension afin de déterminer la pluralité de paramètres de profilage d'hypotension prédisant l'événement d'hypotension futur chez le patient en exécutant le code logiciel de prédiction d'hypotension pour :

réaliser l'analyse de la forme d'onde des données hémodynamiques pour obtenir des paramètres de signes vitaux à partir des données hémodynamiques ajustées ;
dériver des paramètres différentiels basés sur un ou plusieurs des paramètres de signes vitaux ; et
générer des paramètres combinatoires en utilisant un ou plusieurs des paramètres de signes vitaux et/ou un ou plusieurs des paramètres différentiels ;
la pluralité de paramètres de profilage d'hypotension comprenant un ou plusieurs des paramètres de signes vitaux, des paramètres différentiels et des paramètres combinatoires.

13. Système selon la revendication 12,
les paramètres de signes vitaux comprenant un ou plusieurs des paramètres suivants : volume systolique, fréquence cardiaque, respiration et contractilité cardiaque.

14. Système selon la revendication 12 ou selon la revendication 13,
le processeur matériel étant configuré pour exécuter le code logiciel de prédiction d'hypotension pour :

a) dériver les paramètres différentiels basés sur un ou plusieurs des paramètres de signes vitaux en dérivant les paramètres différentiels pour représenter des variations dans le ou les paramètres de signes vitaux par rapport au temps, par rapport à la fréquence, ou par rapport à d'autres paramètres de signes vitaux ; ou
b) générer les paramètres combinatoires en générant les paramètres combinatoires sous la forme d'une combinaison de paramètres de signes vitaux, d'une combinaison de paramètres différentiels ou d'une combinaison d'au moins un paramètre de signe vital et d'au moins un paramètre différentiel.

15. Système selon l'une quelconque des revendications 9 à 14,
le capteur hémodynamique

a) étant un capteur hémodynamique non invasif qui peut être fixé à une extrémité du patient ;
b) étant un capteur hémodynamique peu invasif basé sur un cathéter artériel ; ou
c) produisant les données hémodynamiques sous la forme d'un signal analogique du capteur hémodynamique représentatif de la forme d'onde de la pression artérielle du patient, comprenant en outre, le cas échéant :
un convertisseur analogique-numérique qui convertit le signal analogique du capteur hémodynamique en données hémodynamiques numériques représentatives de la forme d'onde de la pression artérielle du patient.

Fig. 1

Fig. 2

Fig. 3

**Health Monitoring System**

40 — System Processor

System Memory   42

Hypotension Prediction
Software Code  48

Predictive Weighting
Module   50

Hypotension Profiling
Parameters 52

Transformed Hypotension
Profiling Parameters 53

44 — ADC

46 — DAC

54 — User Interface   56   58

Display   12

32

10

36

34

38

# Fig. 4

Fig. 5

RECEIVE ADJUSTED MEAN ARTERIAL PRESSURE (MAP) THRESHOLD FOR HYPOTENSION — 70

RECEIVE SENSED HEMODYNAMIC DATA OF PATIENT — 72

PERFORM WAVEFORM ANALYSIS OF THE HEMODYNAMIC DATA TO DETERMINE HYPOTENSION PROFILING PARAMETERS — 74

GENERATE A SET OF TRANSFORMED HYPOTENSION PROFILING PARAMETERS — 76

DETERMINE, BASED ON THE SET OF TRANSFORMED HYPOTENSION PROFILING PARAMETERS, A RISK SCORE REPRESENTING A PROBABILITY OF A FUTURE HYPOTENSION EVENT FOR THE PATIENT — 78

INVOKE SENSORY ALARM IN RESPONSE TO THE RISK SCORE SATISFYING A PREDETERMINED RISK CRITERION — 80

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20180008205 A1